Europäisches Patentamt

European Patent Office

Office européen des brevets

(11) Publication number: **0 235 419**
**A1**

# (12) EUROPEAN PATENT APPLICATION

(21) Application number: 86301526.9

(22) Date of filing: 04.03.86

(51) Int. Cl.⁴: **B01J 37/08** , B01J 21/08 , C01B 33/12 , C07C 2/86

(43) Date of publication of application:
09.09.87 Bulletin 87/37

(84) Designated Contracting States:
DE FR GB NL

(71) Applicant: **UNION OIL COMPANY OF CALIFORNIA**
**461 South Boylston Street**
**Los Angeles, CA 90017(US)**

(72) Inventor: **Young, Dean Arthur**
**4721 Palm Avenue**
**Yorba Linda California 92686(US)**

(74) Representative: **Jack, Bruce James et al**
**FORRESTER & BOEHMERT**
**Widenmayerstrasse 4/I**
**D-8000 Munchen 22(DE)**

(54) Shock calcined crystalline silica catalysts.

(57) A process for producing a thermally shock calcined crystalline silica comprising (A) precalcining a crystalline silica at a relatively low temperature, (B) very rapidly increasing the temperature of the crystalline silica to a relatively high temperature for a short period of time, and (C) rapidly cooling the crystalline silica. The resulting crystalline silica is catalytically active for hydrocarbon conversion reactions and is particularly selective for the production of para-xylene from a reaction mix of toluene and a methylating agent.

EP 0 235 419 A1

## Shock calcined crystalline silica catalysts

The present invention relates to a crystalline silica and especially to a crystalline silica which is catalytically active and selective in methylation reactions.

A process for selectively producing para-xylene is disclosed in US-A-4,270,017. Para-xylene is selectively prepared by reacting toluene and a methylating agent in the presence of a phosphorus-modified catalyst comprising a silica polymorph intermixed with an inorganic refractory oxide.

US-A-4,325,929 and US-A-4,344,927 relate to a process for hydrothermally preparing a silica polymorph which is described as suitable for use in aromatic alkylation.

US-A-4,362,653 discloses a catalyst composite comprising a silica polymorph and the use of the catalyst in a hydrocarbon reforming process.

Crystalline silicas and their use as alkylation catalysts are disclosed in US-A-4,283,306. This Patent additionally teaches the use of various promoters combined with the catalysts.

Hydrogenation catalysts are disclosed in US-A-4,387,258 which relates to palladium or platinum promoters deposited on a low acidity silica polymorph/silicalite/high silica zeolite.

It is an object of the present invention to provide a crystalline silica for use in catalytically promoting the conversion of hydrocarbons.

Another object of the invention is to provide a catalytically active crystalline silica that is selective for the production of para-xylene.

Yet another object of the invention is to provide a process for the production of para-xylene using catalysts containing said crystalline silica.

Still another object of the invention is to provide a process for producing a crystalline silica.

Other objects and advantages of the invention will be apparent from the following description.

The invention consists in a process for thermally shock calcining a microporous crystalline silica and in silicas produced by such process; in catalysts produced therefrom; and in processes using such catalysts for hydrocarbon conversion reactions, particularly for alkylating an aromatic compound with a $C_1$ to $C_{10}$ hydrocarbon, all as defined in the claims.

Any of the known crystalline silicas may be thermally shock calcined in accordance with the process herein to produce a catalyst which may be used in hydrocarbon conversion reactions.

The preferred crystalline silica composition herein is characterized by pores of uniform diameter of 6 angstroms or less, and even more preferably 5 to 6 angstroms, and is prepared by calcining a crystalline hydrated alkylonium silica prepared by hydrothermal crystallization from a reaction mixture containing as essential reagents, water, amorphous silica and a quarternary ammonium compound, for example tetraethyl ammonium hydroxide, at a pH of at least 10. The compound thus formed is calcined to decompose the alkylonium moieties present. The crystalline silica exhibits no ion exchange properties; however, because of its uniform pore structure it is capable of making size-selective separations of molecular species.

The crystalline silica produced from the above-described mixture has a topological type of tetrahedral framework, which contains a large fraction of five-membered rings of silica-oxygen tetrahedra. The framework comprises a three-dimensional system of intersecting channels which are defined as ten-membered rings of oxygen atoms extending in three directions. Precursor organic nitrogen ions which occupy the intersecting channels are removed by heating or, in the alternative, by extracting with an acid to yield the desired crystalline silica. The resulting void volume occupies approximately 33 percent of the crystal structure, and the three-dimensional channel is wide enough to absorb organic molecules up to about 6 angstroms in diameter. The crystalline silicas, herein, degrade to glass products and dense crystalline silica above about 1500°C - (2732°F).

The crystalline silica employed in this invention is analogous to highly siliceous alkali silicates which form as insoluble compounds during extended hydrothermal digestion. The organic agent, in the form of a nitrogen compound incorporated as a cation during crystallization of the crystalline silicas herein, becomes a source of micropores when eliminated by combustion or extraction. The surfaces of these micropores are relatively free of hydroxyl groups. The isolated hydroxyl groups which are present provide a moderate acidic strength when the crystalline silica is thermally activated. The crystalline silica is a uniquely active solid which is suitable for use as a catalyst component or in catalysts used in hydrocarbon reactions.

The crystalline silica provides not only the required surface for the catalyst precursor, but gives physical strength and stability to the catalyst material. In addition, the crystalline silica has a large surface area upon which the catalyst precursor is deposited.

A crystalline silica suitable for use herein is described in US-A-4,334,927. This crystalline silica is prepared by hydrothermally crystallizing said crystalline silica from a silicate solution containing an organic agent, in combination with a base solution, and an acid solution. Crystallization is effected utilizing high shear mixing. The crystalline silica has as the four strongest d-values of its x-ray diffraction pattern, d = 11.05, d = 0.96, d = 3.82 and d = 3.34. The crystalline silica is designated UCS-3.

Another suitable crystalline silica is described in US-A-4,325,929. The crystalline silica is prepared in accordance with the procedure of US-A-4,344,927 above with the following exception: Crystallization is effected by high shear mixing followed by an unagitated period during the crystallization of said silica. The crystalline silica has as the four strongest d-values of its x-ray diffraction pattern, d = 10.9, d = 4.06, d = 3.83 and d = 3.33. the crystalline silica is designated UCS-4.

A particularly preferred crystalline silica suitable for use herein which exhibits molecular sieve properties is termed silicalite. This material has a specific gravity at 25°C of 1.99 ± 0.05 g/cc as measured by water displacement. After calcination at 600°C in air for 1 hour, silicalite has a specific gravity of 1.70 ± 0.05 g/cc. The mean refractive index of silicalite crystals measured as the synthesized form is 1.48 ± 0.01, while that of the calcined form (600°C in air for 1 hour) is 1.39 ± 0.01.

The x-ray diffraction pattern of silicalite after calcination in air at 600°C for 1 hour has as its six strongest lines or interplanar spacings, d = 11.1 ± 0.2, d = 10.0 ± 0.2, d = 3.85 ± 0.07, d = 3.82 ±0.07, d = 3.76 ± 0.05 and d = 3.72 ± 0.05.

The pore diameter of silicalite is about 6 angstroms and its pore volume is 0.18 cc/g as determined by adsorption. The uniform pore structure of silicalite imparts size-selective molecular sieve properties to the composition. These molecular sieve properties permit the separation of p-xylene from o-xylene, m-xylene and ethylbenzene. A more detailed description of silicalite, including a method of how to prepare the composition, is to be found in US-A-4,061,724.

The crystalline silicas herein exhibit molecular sieve properties characteristic of certain crystalline aluminosilicate compositions, but exhibit substantially none of the ion-exchange properties which are essential to the aluminosilicates commonly referred to as zeolites. The lack of ion-exchange properties in the crystalline silicas herein is due to the crystal lattice structure of the silicas which does not contain alumina as an integral part of said crystal lattice.

Before the crystalline silica is subjected to thermal shock treatment, the crystalline silica is first precalcined at a temperature of from about 370°C - (about 700°F) to about 595°C (about 1100°F), preferably from about 370°C (about 700°F) to about 480°C (about 900°F) for about 30 minutes to about 2 days. Desirably, the thermal shock calcination of the crystalline silica is conducted at a temperature that is at least 165°C (300°F), preferably at least 220°C (400°F) higher than the precalcination temperature. Precalcination facilitates a rapid temperature rise during the subsequent thermal shock calcination by decreasing the heat spent in vaporizing water and desorbing volatile components. Additionally, precalcination avoids the formation of aerosols initiated by the rapid evolution of vapors within the crystalline silica aggregates and particles which may cause fragmentation and fluidization of the crystalline silica during subsequent shock calcination of the silica.

After the precalcination treatment, the crystalline silica is subjected to thermal shock calcination. The thermal shock treatment of the crystalline silica may be carried out in steam, air, ammonia, carbon dioxide, carbon monoxide or any inert atmosphere such as nitrogen, hydrogen, flue gas, argon, helium and mixtures thereof, but it is preferably effected in air. In addition, effluent gases from a combustion chamber may be utilized as a source of direct-fired heat. For maximum efficiency in transferring heat through the crystalline silica, the crystalline silica is reduced to a particle size of less than 3.36 mm (6 mesh), because above about 2.38 or 2.83 mm (7 or 8 mesh), heat transfer becomes a problem and rapid transfer of heat throughout the crystalline silica is difficult to achieve.

During thermal shock calcination, the crystalline silica is subjected to a very rapid increase in temperature wherein the elevated temperature is maintained for a relatively short period of time, because prolonged exposure of the crystalline silica to the relatively high, shock calcination temperature would destroy the original structure of the crystalline silica. Thus, it is critical that the temperature increase very rapidly in the thermal shock calcination of crystalline silicas herein to prevent undesirable fusion and mineralization reactions from occurring. The crystalline silica is heated to a temperature within the range of from about 1040°C (about 1900°F) to about 1260°C (about 2300°F), preferably from about 1095°C (about 2000°F) to about 1205°C (about 2200°F). The crystalline silicas herein are thermally shock calcined by relatively rapidly increasing the temperature to within the range of 1040°C to 1260°C (1900°F to 2300°F) and maintaining the crystalline silica at temperatures within that range for a relatively short period of time, usually from about 0.1 second to about 20 minutes, preferably from about 0.5 second to about 10 minutes, most preferably from 1 second to about 5 minutes. Preferably the thermal shock calcination temperature is increased at a rate of from about 0.5°C (about 1°F) per second to about 111,111°C (about 200,000°F) per second, preferably from about 0.5°C (about 1°F) per second to about 555°C (about 1000°F) per second.

One method of rapidly increasing the temperature of the crystalline silica involves contacting a stream of preheated air with a stream of fluidized crystalline silica powder. The crystalline silica powder typically has a particle size of less than 100 $\mu$m, preferably from about 6 $\mu$m to about 100 $\mu$m, most preferably from about 25 $\mu$m to about 100 $\mu$m, and is fluidized in a flowing gas stream, for example an air stream. A crystalline silica powder having a particle size in this range when mixed with a gas has the characteristics of a fluid when transported through a tube or coil.

A typical apparatus for contacting the air and crystalline silica includes two high-temperature coils connected in series and suspended in a furnace. Air, preheated in the first coil, impinges at a right angle on a stream of fluidized crystalline silica introduced through a tee into the second coil. The crystalline silica is then rapidly cooled, as by introducing a quench stream of cold air into the effluent from the second coil.

Another efficient method of rapidly heating the crystalline silica to the desired temperature is by blending the crystalline silica with a preheated solid silica sand in a sand bath.

Although the invention is not to be held to any particular theory of operation, thermally shock calcining the crystalline silicas herein is believed to alter the crystalline silica surface acidity by the following mechanism: electron-deficient, Lewis-acid sites form when surface hydroxyl groups combine and water is expelled. Crystalline silica surface protonic-acid sites (Bronsted) are eliminated as Lewis-acid sites are formed. The concentration of Lewis-acid sites may decrease as thermal mobility rearranges the crystalline silica and the more active acid sites are eliminated. Thus, the thermal shock calcination of the crystalline silicas herein selectively eliminates the strongest acid sites on the crystalline silica surface resulting in a crystalline silica with slightly reduced catalytic activity but greatly enhanced selectivity. Examples of improved catalyst selectivity are the cracking of hydrocarbons to selectively produce higher proportions of intermediate molecular weight products and in alkylation reactions to the selective production of certain isomers, for example, para-xylene in the reaction of toluene with a methylating agent. The optimal thermal shock calcination temperature for the crystalline silica may vary according to the type of crystalline silica, the desired reaction, and the level of activity and selectivity desired.

After the thermal shock calcination step is completed, it is important to rapidly cool the crystalline silica to a temperature of about 540°C (about 1,000°F) or lower. Rapid cooling of the crystalline silica is necessary because crystalline silicas are excellent thermal insulators and retain the high shock calcination temperatures for a period of time sufficient to cause excess sintering and loss of catalytic activity. The thermally shock calcined crystalline silicas may be cooled, for example, by passing the thermally shock calcined crystalline silica through a tube immersed in a water bath or by flowing the crystalline silica particles over an inclined cooled metal plate or through a rotating cooled tube. Another method of rapidly reducing the temperature of the thermally shock calcined crystalline silicas is by quenching the shock calcined crystalline silica in a liquid medium, such as water.

The shock calcination treatment may be performed on the crystalline silica alone or, if the crystalline silica is to be used as a catalyst in combination with a porous refractory oxide and/or a promoter, then the treatment may be applied to composites containing such components in combination with the crystalline silica. In addition, the crystalline silica treated by shock calcination may be completely or partially cation exchanged, for example, with hydrogen, ammonium, or di-or trivalent metal cations, such as rare earth metal or alkaline earth metal cations for stability purposes or with cations of palladium, platinum, nickel, etc., to provide a hydrogenation component. Other metal cations which may be used include chromium, iron, titanium and zirconium.

The crystalline silicas herein may be used alone, in combination with a refractory oxide, in combination with a promoter or in combination with a refractory oxide and a promoter. It should be noted that the use of refractory oxides and promoters is optional but preferred.

The crystalline silicas may be mixed with an inorganic refractory oxide in the form of a hydrogel or sol such as peptized boehmite alumina or colloidal silica. The inorganic refractory oxides are preferably selected from boehmite alumina, silica hydrosol, colloidal silica and mixtures thereof. Other inorganic refractory oxides include alumina, silica, magnesia, beryllia, zirconia and mixtures thereof.

Normally, the crystalline silica and inorganic refractory oxide are mixed in a weight ratio range of from about 1:10 to about 10:1, preferably from about 1:4 to about 4:1.

In order to provide suitable hydrocarbon conversion, hydrodewaxing, desulfurization and denitrogenation activity, the crystalline silicas herein may be composited with a minor amount of a promoter. The amount of promoter incorporated into the final catalyst is typically from about 0.2 to about 35 weight percent, preferably, from about 0.5 to about 25 weight percent of the catalyst.

For use in hydroconversion reactions, such as hydrodesulfurization and hydrodenitrogenation processes and hydroconversion processes such as hydrocracking, hydroisomerization, reforming etc., a promoter comprising a hydrogenation component is composited with the crystalline silica catalyst. Effective hydrogenation components comprise the Group IIB, Group VIB and Group VIII metals as disclosed in the Periodic Table of Elements, as published by the Sargent-Welch Scientific Company.

Hydrocarbon conversion reactions such as alkylation, isomerization, transalkylation, etc., may be promoted by compositing the crystalline silica catalyst with one or more promoters selected from the Groups IB, IIA, and VA and the rare earth elements of the Periodic Table of Elements as above-described, and preferably compounds of phosphorus, magnesium, boron, antimony, arsenic and mixtures thereof. One especially preferred alkylation promotor is a phosphorus compound.

Representative phosphorus compounds include derivatives of groups represented by the formulae $PX_3$, $RPX_2$, $R_2PX$, $R_3P$, $X_3PO$, $(XO_3)P$, $R_3P=O$, $R_3P=S$, $RPO_2$, $RPS_2$, $RP(O)(OX)_2$, $RP(S)(SX)_3$, $R_2P-(O)OX$, $R_2P(S)SX$, $RP(OX)_2$, $RP(SX)_2$, $ROP(OX)_2$, $RSP(SX)_2$, $(RS)_2PSP(SR)_2$, and $(RO)_2POP(OR)_2$, wherein R is alkyl or aryl and X is hydrogen, alkyl, aryl or halide. These compounds include primary, secondary or tertiary phosphines; tertiary phosphine oxides; tertiary phosphine sulfides; primary and secondary phosphonic acids and their corresponding sulfur derivatives; esters of phosphonic acids; the dialkyl alkyl phosphonates; alkyl dialkyl phosphonates, phosphinous acids, primary, secondary and tertiary phosphites and esters thereof, alkyl dialkyl phosphinites, dialkyl alkyl phosphonites, their esters and sulfur derivatives.

Other suitable phosphorus-containing compounds include the phosphorus halides such as phosphorus trichloride, phosphorus tribromide, phosphorus triiodide, alkyl phosphorodichlorides, dialkyl phosphorochlorides and dialkyl phosphonochloridites. Preferred phosphorus-containing compounds include phosphoric acid, phosphorus acid, and phosphate esters such as trimethylphosphate, ethylphosphate, ethylphosphite, or monophenylphosphate, etc. and mixtures thereof.

Preferred catalysts for alkylation reactions comprise about 5 to about 30 weight percent phosphorus on a support comprising silica or alumina and a shock calcined silicalite, most preferably in a 1:4 to 4:1 weight ratio.

The alkylation process herein may effectively be carried out by contacting an aromatic hydrocarbon and a $C_1$ to $C_{10}$ hydrocarbon with the above-described crystalline silica under alkylation reaction conditions.

The aromatic hydrocarbon is preferably selected from benzene, toluene, xylene, ethylbenzene, phenol, and cresol and mixtures thereof. The preferred aromatic hydrocarbon is toluene.

A wide variety of $C_1$ to $C_{10}$ hydrocarbons may be used to alkylate the aromatic hydrocarbons herein. For example, the $C_1$ to $C_{10}$ alkanes, $C_2$ to $C_{10}$ olefins, as well as $C_1$ to $C_{10}$ alicyclic and alkenyl radicals and various methylating agents may be used.

The shock calcined crystalline silicas may be used alone or in combination with refractory oxides and/or promoters for alkylation reactions. In an especially preferred mode, toluene is selectively alkylated to para-xylene by contacting toluene and a methylating agent with a thermally shock calcined crystalline silica. Optionally, the crystalline silica may contain phosphorus or one or more of the other promoters described herein, preferably in conjunction with an inorganic refractory oxide such as alumina, silica, etc. The reaction is carried out at a temperature of from about 370°C (about 700°F) to about 620°C (about 1,150°F), preferably from about 425°C (about 800°F) to about 540°C (about 1,000°F), and at a pressure of from about atmospheric pressure to about 1,725kPa (about 250 psia), preferably from about 105kPa (about 15 psia) to about 690kPa (about 100 psia). The molar ratio of toluene to methylating agent is normally from about 6:1 to about 1:2, preferably from about 3:1 to about 1:1.

Suitable methylating agents include methanol, methylchloride, methylbromide, dimethyl ether, methylcarbonate, dimethylsulfide, etc. The methylation reaction is accomplished using a weight hourly space velocity (WHSV) of from about 1 to 20, especially from about 2 to about 10. Para-xylene is selectively produced in the reaction; however it should be noted that some ortho-xylene and small amounts of meta-xylene may additionally be produced. Conventional methods may be used to separate the xylene isomers or the undesirable isomers may be converted to paraxylene in an isomerizaiton process. The methylation reaction herein may be carried out as a continuous, semi-continuous or batch type operation, using a fixed or moving type catalyst system utilizing conventional apparatus.

The invention is further illustrated by the following examples which are illustrative of various aspects of the invention and are not intended as limiting the scope of the invention as defined by the appended claims.

## Example I

A crystalline silica (silicalite) is prepared by mixing 1,100 ml of an alkaline solution containing sodium silicate equivalent to the molar proportions: 100 moles of $SiO_2$, 30 moles of $Na_2O$, and 1,390 moles of water, with 530 ml of an acidic solution containing components equivalent to the molar proportions: 16 moles of NaCl, sulfuric acid equivalent to 21 moles $H_2SO_4$, and 710 moles of water. The resulting gel is mixed with an organic solution containing 50 grams of tri-n-propylamine, $(n-C_3H_7)_3$, 49 grams of n-propylbromide, $(n-C_3H_7Br)$ and 94 grams of 2-butanone, $(CH_3COC_2H_5)$. The resulting mixture is stirred and refluxed 24 hours at 82°C - (180°F). Next, the vapor space is pressurized to 4140 kPa (600 psig) with nitrogen and the temperature is increased to 160°C to 166°C (320°F to 330°F) for 30 hours while stirring at about 200 rpm to form a crystalline silica. The solid product is collected by filtration, washed with distilled water and dried at 116°C (240°F).

Next, 1,000 ml of 2.0 M ammonium hydrogen sulfate, $NH_4HSO_4$, is prepared by dissolving 132 grams of ammonium sulfate, $(NH_4)_2SO_4$, in 800 ml of water to form a solution. Next 28 ml of concentrated sulfuric acid, $H_2SO_4$, is added to the solution and the solution is diluted to 1,000 ml with water.

Then, 300 grams of the silicalite prepared in accordance with the procedure described above is added to the ammonium hydrogen sulfate solution and heated at 77°C to 93°C (170°F to 200°F) with stirring for one hour. The acid treated silicalite is collected by filtration, washed with barium acetate, $Ba(C_2H_3O_2)_2$, until the filtrate tests sulfate ($SO_4^=$) free, and dried at 110°C (230°F).

## Example II

Precalcined 70 wt. % Silicalite Bonded with 30 wt. % Alumina

The dried, acid treated silicalite produced in Example I is blended with Catapal S alumina to form a powder mixture containing 70 wt. % silicalite and 30 wt. % alumina. A combustible porosity promoter, equivalent to 10 wt. % of the silicalite composition on a dry weight basis, is added to the mixture by blending said mixture with powdered micro-crystalline cellulose, manufactured by the FMC Corporation.

The above-described powdered mixture is converted into a paste by mulling with sufficient N/10 nitric acid (as 7ml of concentrated nitric acid per 1,000 ml solution). Next, the paste is spread into a thin layer, dried at 150°C (300°F), calcined at 480°C (900°F) for 2 hours, and then granulated into 0.595/2.0 mm (10/30 mesh) aggregates.

## Example III

Shock Calcined 70 wt. % Silicalite Bonded With 30 wt. % Alumina

A portion of the granules produced in accordance with the procedure of Example II is thermally shock calcined by spreading the granules in 3 mm (1/8 inch) layers in zirconia combustion boats. The boats are placed into a preheated Alundum tube. The average rate of temperature increase for the granules is 1°C (2°F)/second in the temperature interval of 845°C to 1180°C (1,550°F to 2,160°F). The temperature is held at 1,180°C to 1,200°C - (2,160°F to 2,190°F) for 4 minutes. Next, the silicalite-containing catalyst is cooled from 1,200°C to 975°C (2,190°F to 1,790°F) at about 0.72°C - (about 1.3°F)/second by drawing the catalyst through a ceramic tube, and then quenched by dumping on a cold steel plate.

## Example IV

Shock Calcined 70 wt. % Silicalite Bonded With 30 wt. % Alumina

A shock calcined, silicalite-containing catalyst is prepared in accordance with the procedure of Example III with the following exception:

The average rate of temperature increase is 1°C (2°F)/second in the temperature interval of 845°C to 1,250°C (1,550°F to 2,280°F) and the temperature is held at 1,250°C to 1,290°C - (2,280°F to 2,350°F) for 3.25 minutes.

## Example V

### Precalcined 70 wt. % Silicalite Bonded With 30 wt. % Silica

The dried, acid treated silicalite (80 grams) produced in Example I is blended with 1.0 gram of micro-crystalline cellulose for 5 minutes. Next, 78 ml of silica and sufficient water are added to the above powdered mixture to form a paste and the resulting mixture is mulled for 5 minutes. Then, the paste is spread into a thin layer, dried at room temperature, granulated into 0.595/2.0 mm (10/30 mesh) aggregates and calcined at 480°C (900°F) for 2 hours.

The calcined granules are slurried in 500 ml of 2.0 M ammonium nitrate and allowed to stand overnight. The granules are collected, as by filtration, washed twice with distilled water, dried at 110°C (230°F) and calcined at 480°C (900°F) for 2 hours.

## Example VI

### Shock Calcined 70 wt. % Silicalite Bonded With 30 wt. % Silica

A precalcined, silicalite-containing catalyst is prepared in accordance with the procedure of Example V to produce a shock calcined catalyst with the following exception:

A portion of the granules produced in accordance with the procedure of Example V is thermally shock calcined by spreading the granules in 3 mm (1/8 inch) layers in zirconia combustion boats. The boats are placed into a preheated Alundum tube. The average rate of temperature increase for the granule is 17°C (30°F)/second. The temperature is held at 1,110°C (2,030°F) for 2 minutes. Next, the silicalite-containing catalyst is quenched by dumping on a cold steel plate.

## Example VII

### Precalcined 10 wt. % $P_2O_5$ on 65 wt. % Silicalite Bonded With 25 wt. % Silica

The dried, acid treated silicalite (26 grams) produced in Example I is blended with 3.5 ml of 85% phosphoric acid and 25 ml of silica to form a mixture and dried at 110°C (230°F). The resulting mixture is calcined at 480°C (900°F) for 2 hours

and then granulated into 10/30 mesh aggregates. The catalyst contained 10 wt. % phosphorus as $P_2O_5$.

## Example VIII

### Shock Calcined 10 wt. % $P_2O_5$ on 65 Wt. % Silicalite Bonded With 25 wt. % Silica

A precalcined, silicalite-containing catalyst is prepared in accordance with the procedure of Example VII to produce a shock calcined catalyst with the following exception:

A portion of the precalcined granules are spread in 3 mm (1/8 inch) layers in zirconia combustion boats. The boats are placed into a preheated Alundum tube. The average rate of temperature increase for the granules is 7°C (12°F)/second. The temperature is held in the temperature interval of from 1,190°C to 1,155°C (2,170°F to 2,110°F) for 3 minutes. Next, the silicalite-containing catalyst is quenched by dumping on a cold steel plate. It should be noted that after shock calcination, the above-described catalyst contains 8.5 wt. % phosphorus as $P_2O_5$.

## Examples IX to XI

Toluene is selectively methylated to para-xylene by feeding toluene and methanol in a molar ratio of 2:1 into a reactor containing the crystalline silica catalysts described in Table 1 below. The toluene and methanol are fed into the reactor at atmospheric pressure, a temperature of 540°C (1,000°F) and a weight hourly space velocity (WHSV) of 4. The catalysts used and results are described and summarized in Table 1 below:

TABLE I

| Ex. | Crystalline Silica Catalyst used | Precalcination $^{\circ}C$ ($^{\circ}F$) Hrs. | | | Shock Calcination $^{\circ}C.$ Min. | | Hours on Stream | Toluene Conversion Wt. % | Selectivity for Xylene Production Wt. % | Xylene Isomers Wt. % P M O | | | Yield p-Isomer Wt. % * |
|---|---|---|---|---|---|---|---|---|---|---|---|---|---|
| IX | Ex.II | 480 (900) | 2 | --- --- | -- | | 3 | 25.9 | 87 | 26 | 49 | 25 | 5.9 |
| X | Ex.III | 480 (900) | 2 | 1180-1200$^a$ | 4 | | 3 | 29.4 | 91 | 63 | 22 | 15 | 16.9 |
| XI | Ex. IV | 480 (900) | 2 | 1250-1290$^b$ | 3.25 | | 3 | 18.7 | 93 | 87 | 8 | 5 | 15.1 |

* The yield is calculated as Wt. % p-xylene based on the toluene feed.

a   2160 - 2190$^{\circ}F$

b   2280 - 2350$^{\circ}F$

The above data prove that the catalysts containing the thermally shock calcined crystalline silica (Examples X and XI) are more selective to the production of para-xylene as compared to the otherwise identical catalyst that does not contain a thermally shock calcined crystalline silica (Example IX). Also, the catalysts of Examples X and XI evidence greater selectivity for xylene production than the catalysts of Example IX.

The features disclosed in the foregoing description and/or in the following claims may, both separately and in any combination thereof, be material for realising the invention in diverse forms thereof.

## Claims

1. A process for treating a microporous crystalline silica which comprises:

(a) precalcining a microporous crystalline silica at a temperature below the temperature to which the crystalline silica is heated in step (b) but sufficiently high to vaporize water and desorb volatile components;

(b) thermally shock calcining the precalcined crystalline silica by rapidly increasing the temperature of said precalcined crystalline silica at a rate between about 0.5°C (1°F)/second and about 111,111°C (200,000°F)/second to a relatively high temperature between about 1,040°C and about 1,260°C (about 1,900°F and about 2,300°F) and maintaining said relatively high temperature for a period of time between about 0.1 second and 20 minutes, said period of time being sufficiently short to avoid substantial sintering; and

(c) cooling the thermally shock calcined crystalline silica at a rate sufficiently rapid to avoid substantial sintering and substantial loss of catalytic activity from said shock calcining.

2. A process according to claim 1, wherein said precalcination temperature is about 220°C (400°F) or more below said relatively high thermal shock calcination temperature.

3. A process according to claim 1 or 2, wherein the temperature of said precalcined crystalline silica in step (b) is increased to said relatively high temperature at a rate of from about 0.5°C (1°F)/second to about 555°C (1,000°F)/second and maintained at said relatively high temperature for about 0.5 second to about 10 minutes.

4. A process according to any one of claims 1 to 3, wherein said precalcined crystalline silica is thermally shock calcined by rapidly increasing the temperature of said precalcined crystalline silica to within the range of from about 1,095°C to about 1,205°C (about 2,000°F to about 2,200°F) and maintaining that temperature for a time between about 1.0 second and about 5 minutes.

5. A process according to any one of claims 1 to 4, wherein said microporous crystalline silica is precalcined at a temperature in the range between about 370°C to about 595°C (about 700°F to about 1,100°F).

6. A process according to any one of claims 1 to 5, wherein said microporous crystalline silica comprises silicalite.

7. A process according to any one of claims 1 to 6, wherein said thermally shock calcined crystalline silica is rapidly cooled to a temperature below about 540°C (1,000°F).

8. A process according to claim 7, wherein said thermally shock calcined crystalline silica is rapidly cooled by passing it through a tube immersed in water.

9. A process according to claim 7, wherein said thermally shock calcined crystalline silica is rapidly cooled by contacting it with a stream of cold air.

10. A process according to claim 7, wherein said thermally shock calcined crystalline silica is rapidly cooled by flowing it over an inclined, cooled metal plate.

11. A process according to claim 7, wherein said thermally shock calcined crystalline silica is rapidly cooled by quenching it in a liquid medium.

12. A process according to claim 7, wherein said thermally shock calcined crystalline silica is rapidly cooled by passing it through a rotating cooled tube.

13. A hydrocarbon conversion process comprising contacting a hydrocarbon feedstock under hydrocarbon conversion conditions with a catalyst comprising a microporous crystalline silica prepared by the process of any one of claims 1 to 12.

14. A process according to claim 13, wherein said catalyst further comprises a hydrogenation component and said hydrocarbon conversion process is selected from hydrodesulfurization, hydrodenitrogenation, hydrocracking, hydroisomerization and reforming.

15. A process according to claim 13 or 14, wherein said catalyst further comprises a promoter selected from phosphorus components, magnesium components, boron components, antimony components, arsenic components and mixtures thereof, and said hydrocarbon conversion process is selected from alkylation, transalkylation and isomerization.

16. A process for alkylating an aromatic hydrocarbon which comprises contacting said aromatic hydrocarbon with a $C_1$ to $C_{10}$ hydrocarbon under alkylation reaction conditions in the presence of an alkylation catalyst comprising a microporous crystalline silica prepared by the process of any one of claims 1 to 12.

17. A process according to claim 16, wherein said aromatic hydrocarbon is selected from benzene, toluene, xylene, ethylbenzene, phenol, cresol and mixtures thereof.

18. A process according to claim 16 or 17, wherein said $C_1$ to $C_{10}$ hydrocarbon is selected from $C_1$ to $C_{10}$ alkanes, $C_2$ to $C_{10}$ olefins, $C_1$ to $C_{10}$ alicyclic radicals and $C_1$ to $C_{10}$ alkenyl radicals.

19. A process according to claim 16, 17 or 18, wherein said alkylation catalyst further comprises an inorganic refractory oxide component and a phosphorus component.

20. A catalyst comprising a microporous crystalline silica prepared by the process of any one of claims 1 to 12.

21. A catalyst according to claim 20, further comprising an inorganic refractory oxide component.

22. A catalyst according to claim 20 or 21, further comprising a hydrogenation component.

23. A catalyst according to claim 20, 21 or 22, further comprising a promoter selected from phosphorus components, magnesium components, boron components, antimony components, arsenic components and mixtures thereof.

24. A catalyst according to any one of claims 20 to 23, further comprising alumina and a phosphorus component.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-1 556 972 (IFP) --- | | B 01 J 37/08 <br> B 01 J 21/08 <br> C 01 B 33/12 <br> C 07 C 2/86 |
| A | FR-A-2 264 055 (MOBIL OIL) --- | | |
| A | EP-A-0 094 138 (STAMICARBON) --- | | |
| A | US-A-3 923 688 (PPG INDUSTRIES) <br><br> * Columns 14,15 * <br><br> ----- | | |
| | | | **TECHNICAL FIELDS SEARCHED (Int. Cl.4)** |
| | | | B 01 J 21/00 <br> B 01 J 37/00 |

The present search report has been drawn up for all claims

| Place of search | Date of completion of the search | Examiner |
|---|---|---|
| THE HAGUE | 07-11-1986 | DEVISME F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82